(19)

**Europäisches Patentamt**
**European Patent Office**
**Office européen des brevets**

(11)  **EP 4 708 312 A1**

(12)  ## EUROPEAN PATENT APPLICATION

(43) Date of publication:
**11.03.2026 Bulletin 2026/11**

(21) Application number: **25177590.4**

(22) Date of filing: **20.05.2025**

(51) International Patent Classification (IPC):
**G16H 20/30** (2018.01)    **A61B 5/11** (2006.01)

(52) Cooperative Patent Classification (CPC):
**G16H 20/30; A61B 5/1118**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **05.09.2024 CN 202411248459**

(71) Applicant: **Anhui Huami Health Technology Co., Ltd.**
**Hefei, Anhui 230088 (CN)**

(72) Inventors:
• **Tang, Qu**
**Hefei, 230088 (CN)**

• **Liu, Chang**
**Hefei, 230088 (CN)**
• **Liu, Yingfei**
**Hefei, 230088 (CN)**
• **Meng, Yabo**
**Hefei, 230088 (CN)**
• **Wang, Kongqiao**
**Hefei, 230088 (CN)**

(74) Representative: **Dai, Simin**
**Reyda IP**
**A073**
**157, Quai du Président Roosevelt**
**92130 Issy-les-Moulineaux (FR)**

(54)  **METHODS AND APPARATUSES FOR ACTION QUALITY EVALUATION, STORAGE MEDIA AND ELECTRONIC DEVICES**

(57)  The embodiments of the present disclosure provide methods and apparatuses for action quality evaluation, storage media and electronic devices, where the method for action quality evaluation includes: acquiring motion data of a user collected during a fitness session; determining a value of at least one movement evaluation metric of the user based on the motion data; and determining an action quality evaluation result of the user based on the value of the at least one movement evaluation metric. With the scheme for action quality evaluation provided by the embodiments of the present disclosure, the action quality evaluation result determined based on the value of one or more action evaluation metrics can be utilized to characterize the action quality of the user, which is easier for the user to understand, and thus meets the needs of the user.

FIG. 2

EP 4 708 312 A1

**Description**

TECHNICAL FIELD

The present disclosure relates to computer technologies, and more particularly, to methods and apparatuses for action quality evaluation, storage media and electronic devices.

BACKGROUND

With the escalating intelligence of portable electronic devices, an increasing number of users are using portable electronic devices as exercise aids. For example, users utilize portable electronic devices to record exercise information during fitness activities such as running, swimming, or strength training, and one or more performance parameters of the fitness activities are measured based on the exercise information recorded by the portable electronic devices. However, the performance parameters of the fitness activities often prove to be overly abstract and cannot meet the needs of the users.

SUMMARY

In view of the above, the embodiments of the present disclosure provide methods and apparatuses for action quality evaluation, a storage medium and an electronic device.

According to a first aspect of the present disclosure, a method for action quality evaluation is provided, including:

> obtaining motion data of a user collected during a fitness session;
> determining a value of at least one action evaluation metric of the user according to the motion data; and
> determining an action quality evaluation result of the user according to the value of the at least one action evaluation metric;
> where the at least one action evaluation metric includes at least one of:
>
>> action consistency, for evaluating at least one of amplitude consistency or completion time consistency of a plurality of fitness actions performed by the user;
>> action stability, for evaluating a degree of jitter or wobbling of one or more fitness actions performed by the user;
>> action continuity, for evaluating a continuity degree of one or more fitness actions performed by the user;
>> action variability, for evaluating an intensity variability of multiple fitness actions performed by the user; or
>> force control, for evaluating force exertion condition during one or more fitness actions performed by the user.

In conjunction with any implementation provided in the present disclosure, determining the value of the at least one action evaluation metric of the user according to the motion data includes:

> determining metric feature data for each of at least one fitness action performed by the user according to the motion data; and
> obtaining the value of the at least one action evaluation metric for the user according to the metric feature data for each of the at least one fitness action.

In conjunction with any implementation provided in the present disclosure, determination of the metric feature data for each fitness action is independent of an exercise type of the each fitness action.

In conjunction with any implementation provided in the present disclosure, determination of the metric feature data for each of the at least one fitness action includes performing data reconstruction processing on the motion data, so as to mitigate or eliminate an influence of the exercise type, or a same algorithm or model can be used without taking into the exercise types of the fitness actions into consideration.

In conjunction with any implementation provided in the present disclosure, the metric feature data for each of the at least one fitness action is obtained by performing singular value decomposition processing on the motion data.

In conjunction with any implementation provided in the present disclosure, determining the value of the at least one action evaluation metric of the user according to the motion data includes: determining the value of at least one action evaluation metric without taking an exercise type of at least one fitness action performed by the user into consideration.

In conjunction with any implementation provided in the present disclosure, determining the value of the at least one action evaluation metric of the user according to the motion data includes: performing data reconstruction processing on the motion data, so as to mitigate or eliminate an influence of an exercise type.

In conjunction with any implementation provided in the present disclosure, determining the value of the at least one action evaluation metric of the user according to the motion data includes: performing singular value decomposition processing on the motion data.

In conjunction with any implementation provided in the present disclosure, determining the value of the at least one action evaluation metric of the user according to the motion data includes:

> obtaining at least one motion data segment according to the motion data, each motion data segment includes motion data corresponding to at least one fitness action;
performing data reconstruction processing on each of the at least one motion data segment to obtain at least one reconstructed data segment; and obtaining the value of the at least one action evaluation metric of the user according to the at least one reconstructed data segment.

In conjunction with any implementation provided in the present disclosure, the reconstructed data segment includes at least one of virtual principal axis data or virtual secondary axis data, where the virtual principal axis data is used for representing a signal response to primary motion of the user, and the virtual secondary axis data is used for representing a signal response to sources other than the primary motion.

In conjunction with any implementation provided in the present disclosure, obtaining the value of the at least one action evaluation metric of the user according to the at least one reconstructed data segment includes: determining a value of at least one first action evaluation metric for one or more fitness actions performed by the user according to virtual principal axis data in the reconstructed data segment, the at least one first action evaluation metric including at least one of action consistency, action stability, action continuity, action variability or action regularity.

In conjunction with any implementation provided in the present disclosure, obtaining the value of the at least one action evaluation metric of the user according to the at least one reconstructed data segment includes: determining a value of at least one second action evaluation metric for one or more fitness actions performed by the user according to virtual secondary axis data in the reconstructed data segment, the at least one second action evaluation metric including at least one of action stability or force control.

In conjunction with any implementation provided in the present disclosure, the action quality evaluation result of the user includes at least one of:

a statistical result of the value of the at least one action evaluation metric of the user;
evaluative description of the at least one action evaluation metric of the user; or an action improvement suggestion for the user.

According to a second aspect of the present disclosure, another method for action quality evaluation is provided, including:

obtaining motion data of a user collected during a fitness session;
obtaining at least one motion data segment according to the motion data, each motion data segment includes motion data corresponding to at least one fitness action;
performing data reconstruction processing on each of the at least one motion data segment to obtain at least one reconstructed data segment; and obtaining an action quality evaluation result of the user according to the at least one reconstructed data segment.

In conjunction with any implementation provided in the present disclosure, the data reconstruction processing is used to mitigate or eliminate an influence of an exercise type of the fitness action.

In conjunction with any implementation provided in the present disclosure, the data reconstruction processing includes singular value decomposition processing.

In conjunction with any implementation provided in the present disclosure, the reconstructed data segment includes at least one of virtual principal axis data or virtual secondary axis data, where the virtual principal axis data is used for representing a signal response to primary motion of the user, and the virtual secondary axis data is used for representing a signal response to sources other than the primary motion.

In conjunction with any implementation provided in the present disclosure, obtaining the action quality evaluation result of the user according to the at least one reconstructed data segment includes: determining a value of at least one action evaluation metric for each of the at least one motion data segment according to the at least one reconstructed data segment; and determining the action quality evaluation result of the user according to the value of the at least one action evaluation metric for each of the at least one motion data segment.

In conjunction with any implementation provided in the present disclosure, obtaining the action quality evaluation result of the user according to the at least one reconstructed data segment includes:
determining a value of at least one first action evaluation metric for one or more fitness actions performed by the user according to virtual principal axis data in the reconstructed data segment, and the at least one first action evaluation metric includes at least one of action consistency, action stability, action continuity, action variability or action regularity.

In conjunction with any implementation provided in the present disclosure, obtaining the action quality evaluation result of the user according to the at least one reconstructed data segment includes:
determining a value of at least one second action evaluation metric for one or more fitness actions performed by the user according to virtual secondary axis data in the reconstructed data segment, and the at least one second action evaluation metric includes at least one of action stability or force control.

According to a third aspect of the present disclosure, an apparatus for action quality evaluation is provided, including: at least one unit or module, configured to execute the method for action quality evaluation in any possible implementation of any aspect of the present disclosure.

In some embodiments, the at least one unit or module includes:

a motion data acquisition module, configured to acquire motion data of a user collected during a fitness session;

a metric value determination module, configured to determine a value of at least one action evaluation metric of the user based on the motion data; and

an evaluation result determination module, configured to determine an action quality evaluation result of the user according to the value of the at least one action evaluation metric.

In some other embodiments, the at least one unit or module includes:

a motion data acquisition module, configured to acquire motion data of a user collected during a fitness session;

a motion data segment determination module, configured to obtain at least one motion data segment based on the motion data, where each motion data segment includes motion data corresponding to at least one fitness action;

a reconstructed data segment determination module, configured to perform data reconstruction processing on each of the at least one motion data segment to obtain at least one reconstructed data segment; and

a second assessment result determination module, configured to obtain an action quality evaluation result of the user based on the at least one reconstructed data segment.

According to a fourth aspect of the present disclosure, a computer-readable storage medium is provided, with machine-readable instructions stored thereon, and when the machine-readable instructions are executed by a processor, the processor is caused to implement the method for action quality evaluation in any implementation of any aspect of the present disclosure.

According to a fifth aspect of the present disclosure, an electronic device is provided, including:

a processor; and

a memory for storing instructions executable by the processor;

where the processor is configured to execute the method for action quality evaluation in any implementation of any aspect of the present disclosure.

The technical solutions provided in the embodiments of the present disclosure can achieve the following beneficial effects:

In the methods and apparatuses for action quality evaluation, the storage medium and the electronic device provided in the embodiments of the present disclosure, the motion data of the user collected during the fitness session (i.e., fitness activity, fitness process) is firstly obtained, and then, the value of the at least one action evaluation metric of the user is determined according to the motion data. Finally, the action quality evaluation result of the user is determined according to the value of the at least one action evaluation metric. With the scheme for the action quality evaluation provided in the embodiments of the present disclosure, the action quality evaluation result is determined based on the values of one or more action evaluation metrics and is used for characterizing the action quality of the user, which is easier for the user to understand, thereby meeting the user's needs.

It should be understood that the above mentioned general description and the following detailed description are merely illustrative and explanatory, and shall not impose any limitations on the present disclosure.

BRIEF DESCRIPTION OF DRAWINGS

Drawings are provided to better understand the present disclosure and are not intended to limit the present disclosure.

To illustrate the technical solutions in one or more embodiments of the present disclosure or related technologies more clearly, the following provides a brief introduction to the drawings used in the description of the embodiments and related technologies:

FIG. 1 is a structural schematic diagram of a system for action quality evaluation according to an exemplary embodiment of the present disclosure;

FIG. 2 is a flowchart of a method for action quality evaluation according to an exemplary embodiment of the present disclosure;

FIG. 3 is an exemplary user interface for illustrating an action quality evaluation result according to an exemplary embodiment of the present disclosure;

FIG. 4 is a flowchart of another method for action quality evaluation according to an exemplary embodiment of the present disclosure;

FIG. 5 is a structural schematic diagram of an apparatus for action quality evaluation shown according to an exemplary embodiment of the present disclosure;

FIG. 6 is a structural schematic diagram of another apparatus for action quality evaluation according to an exemplary embodiment of the present disclosure;

FIG. 7 is a structural schematic diagram of an electronic device according to an exemplary embodiment of the present disclosure.

DETAILED DESCRIPTION

Exemplary embodiments will be described herein in detail, and examples thereof are shown in the accompanying drawings. In the following description, if the drawings are referred to, unless otherwise specified, a same reference number in different drawings represents a same or similar elements.

In order to enhance the effect of workout, users typically desire to know their performance during the workout or fitness sessions. Therefore, users can wear, hold or attach portable electronic devices during the workout process, and utilize at least one sensor of the portable electronic devices to measure physiological and/or motion data of the users during workout. For example, parameters such as speed, acceleration and/or the like are measured. The portable electronic devices may be wearable devices for example, which are worn on the body parts of users, such as wrist, ear, eye, head, neck, torso, waist, foot and/or the like, or may be portable devices attached to clothing, jewelry or other electronic devices. The portable electronic devices may collect one or more types of physiological data of the users during the workout or fitness sessions, such as heart rate, blood oxygen, blood pressure, body temperature and/or the like, or collect the motion data of the users, or collect the environmental data of the environment where the fitness sessions of the users are conducted. Then, the collected one or more types of data is analyzed and processed to obtain one or more motion performance indicators of the users. The one or more motion performance indicators are provided to the users, which helps the users understand their own motion performance and improve or strengthen one or some aspects, thereby improving the effect of workout.

The embodiments of the present disclosure provide a system for action quality evaluation, which may obtain motion data of the user collected during a workout or fitness session, and determine a value of at least one action evaluation metric of the user according to the motion data. Subsequently, an action quality evaluation result of the user is determined according to the value of the at least one action evaluation metric.

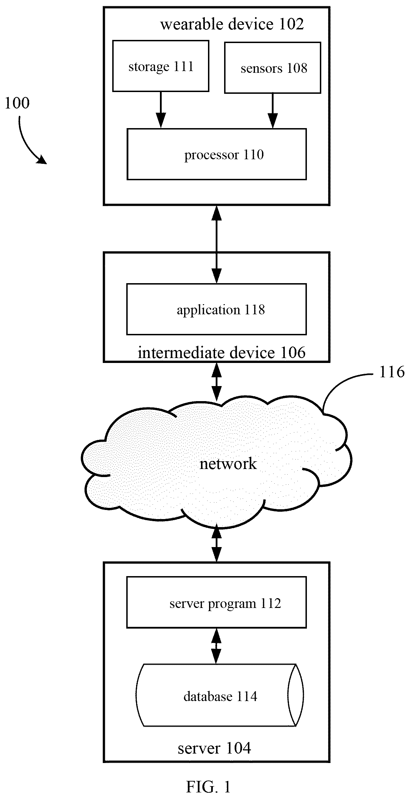

Referring to FIG. 1, which shows a structural schematic diagram of the system for action quality evaluation applicable for the embodiments of the present disclosure. The system may include a wearable device 102, a server 104 and an intermediate device 106.

The wearable device 102 is a computing device configured to be worn by an individual during operation. The wearable device 102 may be implemented as a wristwatch, wristband, bracelet, armband, leg-band, leg-ring, etc., or in the form of other types of wearable devices. The wearable device 102 includes one or more sensors 108 for detecting the motion data of the user wearing the wearable device 102.

In some examples, the sensors 108 may include a motion sensor, which is configured to measure the motion data of the user. For example, the sensors 108 may include one or more of an accelerometer, a gyroscope, a magnetometer, a speed sensor, a displacement sensor, or other types of sensors, or a combination thereof. The motion parameters are measurable parameters associated with the movement of the user wearing the wearable device 102. In some examples, the motion data collected during various fitness sessions of the user may include one or more of acceleration, velocity, displacement, Euler angles, or other types of motion data. The sensors 108 may continuously, intermittently, or otherwise periodically collect the motion data of the user.

In some other examples, the sensor 108 may include one or more physiological sensors for measuring one or more physiological parameters of the user, such as heart rate, body temperature, emotion, stress, blood oxygen, blood pressure, blood glucose, or other types of blood analyte metrics or the like.

In some other examples, the sensors 108 may include one or more environmental sensors for measuring the environmental data of the environment where the user is currently located. The environmental data may include, for example, one or more of data about positioning, indoor or outdoor, ambient temperature, ambient humidity, air quality, barometric pressure, altitude, etc.

The wearable device 102 further includes a processor 110 and a memory 111. The memory 111 stores application programs or other executable instructions. The processor 110 is configured to run the application programs or other executable instructions to process the motion data generated based on the motion signals collected by the sensors 108.

The wearable device 102 may further include input and/or output components, such as one or more of a display, an audio output component, a tactile output component, and/or other types of output components. The display may include one or more of a touch sensor, a force sensor, a pressure sensor, etc., which is not limited herein.

The server 104 may include a hardware server (e.g., a server), a software server (e.g., a web server), and/or a virtual server. The server program 112 is software for detecting movement conditions of the user wearing the wearable device 102.

The server program 112 may access the database 114 on the server 104 to perform at least some functions of the server program 112. The database 114 is a database or other types of data storage for storing, managing, or otherwise providing data for delivering the functions of the server program 112. For example, the database 114 may be a relational database management system, an object database, an XML database, a configuration management database, a management information repository, one or more flat files, other appropriate non-transient storage mechanisms, or a combination thereof.

The intermediate device 106 is configured for facilitating communication between the wearable device 102 and the server 104. The intermediate device 106 may be a computing device, such as a mobile terminal (e.g., a smartphone, a tablet, a laptop, etc.) or other computers (e.g., a desktop computer or other non-mobile computers). Alternatively, the intermediate device 106 may be a network hardware, such as a router, a switch, a load balancer, or other network devices, or a combination thereof. Alternatively, the intermediate device 106 may be other types of network connection devices. For example, the intermediate device 106 may be a networked power charger for the wearable device 102.

For example, depending on the specific implementations of the intermediate device 106, the intermediate device 106 may run the application program 118. The application program 118 configures the intermediate device 106 to send data to the wearable device 102 or receive data from the wearable device 102, and/or to transfer data to and from the server 104. Moreover, the application program 118 be configured to receive commands from the user of the intermediate device 106 in response to user operations. For example, in the case that the intermediate device 106 is a computing device with a touch screen display, the user of the intermediate device 106 may issue commands by touching a portion of the display corresponding to a user-interface element of the application program.

In some implementations, the client device is authorized to access the server program 112. In some examples, the client device may be a mobile device, such as a smartphone, a tablet, a laptop, etc. In some other examples, the client device may be a desktop computer or other non-mobile computers. The client device may run a client application program to communicate with the server program 112. For example, the client application program may be a mobile application capable of accessing some or all of the functions and/or data of the server program 112. In some examples, the client device may communicate with the server 104 via the network 116. In some of such implementations, the client device may be the intermediate device 106.

In some implementations, the intermediate device 106 receives data from the wearable device 102 with a short-range communication protocol. For example, the short-range communication protocol may be Bluetooth®, Bluetooth® Low Energy, infrared, Z-Wave, ZigBee, or other types of protocols, or a combination thereof. The intermediate device 106 sends the data received from the wearable device 102 to the server 104 via the network 116. For example, the network 116 may be a local area network, a wide area network, a machine-to-machine network, a virtual private network, or other public or private networks. The network 116 may use a remote communication protocol, such as Ethernet, TCP, IP, power-line communication, Wi-Fi, GPRS, GSM, CDMA, or other types of protocols, or a combination thereof.

In some implementations, the intermediate device 106 may be omitted. For example, the wearable device 102 is configured to communicate directly with the server 104 via the network 116. The direct communication between the wearable device 102 and the server 104 via the network 116 may include for example the use of a remote, low-power system or other communication mechanisms.

In the embodiments of the present disclosure, the aforementioned method for action quality evaluation may be performed by any one of the wearable device, the intermediate device (such as a mobile terminal), or the server. Alternatively, the aforementioned method for action quality evaluation may be performed cooperatively by at least two of the wearable device, the intermediate device, and the server.

In some examples, the wearable device obtains the motion data of the user collected by at least one sensor during the fitness session, and sends the motion data to the intermediate device. The intermediate device analyzes and processes the motion data to obtain the value of at least one action evaluation metric of the user, and sends the value of the at least one action evaluation metric to the server. The server determines the action quality evaluation result of the user based on the value of at least one action evaluation metric, and then sends the action quality evaluation result to at least one of the intermediate device or the wearable device for output to the user.

In some other examples, the wearable device obtains the motion data of the user collected during the fitness session by at least one sensor and then sends the data to the intermediate device or the server. The intermediate device or the server analyzes and processes the motion data to obtain the value of at least one action evaluation metric of the user, and determines the action quality evaluation result of the user based on the value of the action evaluation metric. Subsequently, the intermediate device or the server sends the action quality evaluation result to the wearable device for output to the user.

In some other examples, the processor of the wearable device obtains the motion data of the user collected by at least one sensor during the fitness sessions, analyzes and processes the motion data to obtain the value of at least one action evaluation metric of the user, determines the action quality evaluation result of the user based on the value of at least one action evaluation metric, and either outputs the action quality evaluation result to the user, or sends the action quality evaluation result to the intermediate device, so that the intermediate device outputs the action quality evaluation result for the user.

It should be noted that the foregoing description is merely illustrative, and is intended to enable those skilled in the art to better understand the technical solutions of the embodiments of the present disclosure. The present disclosure does not impose any specific limitation on the entity that executes the procedures of the aforementioned method for action quality evaluation.

A detailed description of the method for action quality evaluation in some exemplary embodiments of the present disclosure is provided below in conjunction with the accompanying drawings.

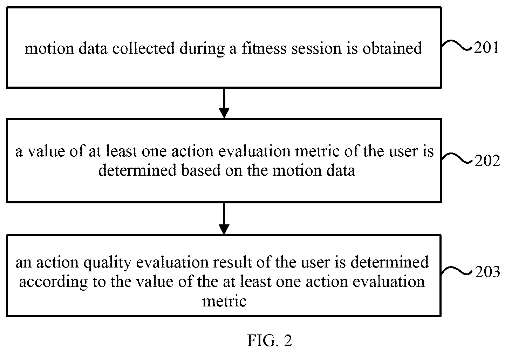

FIG. 2 is a flowchart of the method for action quality evaluation according to an exemplary embodiment of the present disclosure. As shown in FIG. 2, the method in the exemplary embodiment includes 201 to 203. The method may be executed by the system for action quality evaluation to evaluate the action quality of the user during fitness session such as strength training or the like.

At 201, motion data collected during a fitness session is obtained.

The motion data may include at least one of acceleration data, angular velocity data, velocity data, displacement data, Euler angle data, or geomagnetic sensor data.

In some examples, the system for action quality evaluation may include at least one of an accelerometer, a gyroscope, or a geomagnetic sensor. The accelerometer may be configured to collect acceleration data, velocity data, or displacement data. The gyroscope may be configured to collect angular velocity data or Euler angle data. The geomagnetic sensor may be configured to collect data about the earth's magnetic field. Alternatively, the system for action quality evaluation may include other types of motion sensors, which is not limited herein.

In some examples, during the fitness session, the user may perform a plurality of fitness actions, and the plurality of fitness actions may belong to a same type of exercise, or belong to different types of exercises. For instance, the user may start with one or more types of warm-up exercises such as jumping jacks and/or the like; and then the user may move on to perform one or more types of strength training exercises, such as rowing, deadlifting and/or the like, and finally, the user may do one or more types of stretching exercises to complete the fitness session.

In some instances, after obtaining raw data collected by the motion sensor, at least a portion of the raw data is processed to obtain at least one of velocity data, displacement data, Euler angle data or the like. The types of data included in the motion data are not limited herein.

The processor may obtain the motion data over a period of time, or obtain the motion data within a window of a fixed length.

At 202, a value of at least one action evaluation metric of the user is determined based on the motion data.

The at least one action evaluation metric includes at least one of action consistency, action stability, action continuity, action variability, or force control.

The action consistency is used for evaluating at least one of amplitude consistency or completion time consistency of a plurality of fitness actions performed by the user. Generally, the more consistent the actions are, the higher the action quality is.

The action stability is used for evaluating a degree of jitter or wobbling of one or more fitness actions performed by the user, and may be used to measure a deviation of the user in a non-force-exertion direction. It may refer to a degree of jitter or wobbling of at least a portion of the user's body in terms of displacement or angle in one or more directions. For example, the action stability refers to shifting, swaying, jittering, tilting or the like of at least a portion of the user's body in the non-force-exertion direction. Generally, the more stable the action is, the higher the action quality is.

The action continuity is used for evaluating coherence of one or more fitness actions carried out by the user, and may be used to measure whether there is any abrupt or unnecessary pause or interruption during the execution of one or more fitness actions. Generally, the smoother the action is, the better the action quality is.

The action variability is used for evaluating intensity fluctuations of a plurality of fitness actions carried out by the user, and may be used to measure whether the user reaches exhaustion or fatigue.

The force control is used for evaluating force exertion patterns during one or more fitness actions performed by the user, and may be used to measure a force exertion condition of the user during the execution of fitness actions. For example, the force control is used to determine whether there is a sudden force exertion or a sharp change in the exerted force, or whether the user relies on inertia to complete the actions, or the like.

In some embodiments, if the fitness actions carried out by the user are symmetrical actions, or, if the user has sensors attached to different body parts, the at least one action evaluation metric may further include action balance, which is used for evaluating a left-right balance degree of the fitness actions performed by the user. In some other embodiments, the at least one action evaluation metric may further include action regularity, which is used for evaluating whether one or more fitness actions performed by the user are standard. Optionally, the at least one action evaluation metric may include other types of metrics, which is not limited herein.

The method for action quality evaluation in the embodiments of the present disclosure is applicable to various types of exercises (or referred to as exercise types, action types, or types of fitness actions), such as strength training (e.g., squats, sit-ups, jumping jacks, burpees, push-ups, pull-ups, presses, rows, raises, deadlifts, etc.), running, swimming, etc.

In some embodiments, the motion data is segmented to obtain a plurality of motion data segments, where each motion data segment may include one or more fitness actions. For example, the motion data of two fitness actions performed by the user may be segmented into a motion data segment corresponding to fitness action 1 and a motion data segment corresponding to fitness action 2. Subsequently, by processing each of the multiple motion data segments, the values of the action evaluation metrics of a plurality of fitness actions corresponding to the multiple motion data segments are obtained.

In some embodiments, cycle detection may be performed on the obtained motion data to segment the motion data into a plurality of motion data segments, where each motion data segment may include one or more cycles. For instance, based on the obtained motion data, one or more of the acceleration data, velocity data, displacement data or the like is subjected to waveform analysis, such as at least one of peak detection, trough detection, zero-point detection or the like, and an action cycle is determined to classify different fitness actions.

In some other embodiments, one or more types of pre-processing may be performed on the obtained motion data, such as one or more of filtering, denoising, and data conversion processing. In an optional example, a coordinate system conversion processing is performed on the obtained motion data to convert the motion data from the device coordinate system to the world coordinate system. The coordinate system conversion processing may be implemented with any suitable attitude analysis algorithm or model, which is not limited herein.

In some embodiments, determining the value of the at least one action evaluation metric of the user based on the motion data includes: determining the value of the at least one action evaluation metric without taking an exercise type of at least one fitness action performed by the user into consideration; or performing data reconstruction processing on the motion data, so as to mitigate or eliminate an influence of the exercise type; or performing singular value decomposition processing on the motion data.

In some embodiments, in order to mitigate or eliminate the influence of the type of fitness actions (i.e., the exercise type) on the determination of the action evaluation metrics of fitness actions, so that the processing of the motion data is independent of a specific type of fitness actions and be universally applicable to a variety of types of fitness actions, after segmenting the motion data to obtain a plurality of motion data segments, data reconstruction processing may be performed on each of the plurality of motion data segments to obtain a plurality of reconstructed data segments. Subsequently, based on the obtained plurality of reconstructed data segments, the values of the action evaluation metrics of the plurality of fitness actions corresponding to the plurality of motion data segments are determined.

In some embodiments, the motion data may include data from more than two axes, such as acceleration data in three axes, acceleration data and angular velocity data in six axes, or accelerometer data, gyroscope data and magnetometer data in nine axes or the like. The data reconstruction processing may be performed on each of the plurality of motion data segments in the following manner: for each motion data segment, data decomposition, data analysis, or coordinate transformation processing may be performed on the motion data segment by using an algorithm such as SVD (Singular Value Decomposition) algorithm, IDA (Independent Component Analysis) algorithm, or PCA (Principal Component Analysis) algorithm or the like, to obtain multi-axis processed data; and then, data reconstruction is performed on the obtained multi-axis processed data to obtain a reconstructed data segment. The data reconstruction processing separates the primary signal response and the secondary signal response from the motion data segment, or extract the primary data features and the secondary data features from the motion data segment, thereby facilitating subsequent determination of the values of the action evaluation metrics.

In some optional embodiments, the reconstructed data segment may include a primary signal response or a signal response to primary motion of the user, and/or, include a secondary signal response or a signal response to other sources other than the primary motion such as noise. For instance, the reconstructed data segment may include at least one of virtual principal axis data or virtual secondary axis data. The virtual principal axis data and the virtual secondary axis data are data on virtual axes constructed during the data reconstruction processing, corresponding to signal responses to the primary motion of the user and signal responses to other sources, respectively. In this way, the value of at least one action evaluation metric of the user is determined based on at least one of the virtual principal axis data or the virtual secondary axis data. In an example, a value of at least one first action evaluation metric for one or more fitness actions performed by the user is determined according to the virtual principal axis data in the reconstructed data segment, the at least one first action evaluation metric including at least one of action consistency, action stability, action continuity, action variability or action regularity. In another example, a value of at least one second action evaluation metric for one or more fitness actions performed by the user is determined according to the virtual secondary axis data in the reconstructed data segment, the at least one second action evaluation metric including at least one of action stability or force control.

In some embodiments, singular value decomposition processing may be performed on the motion data segment to obtain multi-axis processed data, and then, data of the first two or more axes with a stronger signal response in the multi-axis processed data is reconstructed to obtain the virtual principal axis data, and data of the last one or more axes with a weaker signal response in the multi-axis processed data is reconstructed to obtain the virtual secondary axis data. In this way, the obtained virtual principal axis data is associated with a main movement of the user, and reconstructed to include a component corresponding to larger singular values. The virtual principal axis data is used to represent the signal response to the primary motion of the user (or represent a major trend of change or energy concentrations in the motion data). The virtual secondary axis data is reconstructed to include a component corresponding to smaller singular values. The virtual secondary axis data is used to represent the signal response to other sources other than the primary motion of the user (or represent a relatively small variation in the motion data caused by noise, minor distractions, or other components of movement that are less significant compared to the main movement).

In some embodiments, each of the multiple motion data segments may include at least one of acceleration data, velocity data, displacement data, or Euler angle data, or may further or alternatively include other types of data. Correspondingly, the data reconstruction processing may be performed on each type of motion data included in the motion data segment separately to obtain the reconstructed data segment corresponding to each type of motion data, or, the data reconstruction processing may be performed on only a portion of the motion data with a particular type to obtain the reconstructed data segment corresponding to the particular type of motion data, and the reconstructed data segment corresponding to other types of motion data may be obtained based on the reconstructed data segment corresponding to the particular type of motion data, but the present disclosure does not limit hereto.

In an example, in response to the motion data segment including at least one of acceleration data or angular velocity data, the data reconstruction processing is performed on at least one of the acceleration data or the angular velocity data included in the motion data segment to obtain at least one of a reconstructed acceleration data segment or a reconstructed angular velocity data segment. The reconstructed acceleration data segment may include at least one of virtual principal axis acceleration data and virtual secondary axis acceleration data. The reconstructed angular velocity data segment may include at least one of virtual principal axis angular velocity data and virtual secondary axis angular velocity data.

In another example, in response to the motion data segment including at least one of velocity data or Euler angle data, the data reconstruction processing is performed on at least one of the velocity data or the Euler angle data included in the motion data segment to obtain at least one of a reconstructed velocity data segment or a reconstructed Euler angle data segment. The reconstructed velocity data may include at least one of virtual principal axis velocity data or virtual secondary axis velocity data. The reconstructed Euler angle data segment may include at least one of virtual principal axis Euler angle data or virtual secondary axis Euler angle data.

In another example, in response to the displacement data being included in the motion data segment, the data reconstruction processing is performed on the displacement data included in the motion data segment to obtain a reconstructed displacement data segment. The reconstructed displacement data segment may include at least one of virtual principal axis displacement data or virtual secondary axis displacement data.

In another example, after performing data reconstruction processing on the motion data segment to obtain at least one of the reconstructed acceleration data segment or the reconstructed angular velocity data segment, at least one of a reconstructed velocity data segment or a reconstructed displacement data segment may be further obtained based on the reconstructed acceleration data segment. Alternatively, a reconstructed Euler angle data segment may be further obtained based on the reconstructed angular velocity data segment. The embodiments of the present disclosure do not limit the manner of obtaining each reconstructed data segment.

In addition, the foregoing embodiments are described in terms of performing data reconstruction processing on each of the plurality of motion data segments separately as an example, where the object of the data reconstruction processing is a single motion data segment. In some other embodiments, the data reconstruction processing is performed on two or more motion data segments. e.g., the data reconstruction processing may be performed jointly on two or more adjacent motion data segments, or the data reconstruction processing is performed on a motion data segment with reference to at least one adjacent motion data segment.

In some embodiments, after the data reconstruction processing is performed on each of the plurality of motion data segments to obtain multiple reconstructed data segments, for each reconstructed data segment, metric feature data associated with at least one action evaluation metric for one or more fitness actions corresponding to the reconstructed data segment is calculated, and then, the value corresponding to the at least one action evaluation metric is determined respectively based on the calculated metric feature data corresponding to the at least one action evaluation metric.

In some embodiments, some or each of the plurality of motion data segments may correspond to one fitness action. Correspondingly, the value of at least one action evaluation metric corresponding to the fitness action is obtained based on the reconstructed data segment corresponding to the motion data segment.

In some other embodiments, some or each of the plurality of motion data segments may correspond to two or more fitness actions. In this case, the value of the at least one action evaluation metric corresponding to the two or more fitness actions may be obtained, where the at least one action evaluation metric is used for evaluating the overall performance of the two or more fitness actions. Alternatively, the value of the at least one action evaluation metric corresponding to each of the two or more fitness actions may be obtained. Alternatively, both of the above (i.e., the overall metric value (i.e., the value of a metric) for the two or more fitness actions as well as the metric value for each of the two or more fitness actions) may be obtained.

In some other embodiments, during the data segmentation processing, the motion data for each fitness action may be obtained, and then, at least two fitness actions satisfying a specific condition may be divided into a group. For example, a similarity between at least two neighboring fitness actions may be calculated based on the motion data corresponding to the at least two neighboring fitness actions. If the resulted similarity is greater than a preset similarity threshold or meets other conditions indicating that these two neighboring fitness actions are more likely to be of a same type of fitness actions, the at least two neighboring fitness actions may be divided into a group. In another example, user input may be received, such as input for selecting, starting, or stopping a certain type of exercise, input for indicating a change of fitness action or action type, input for indicating a switch of a grouping or the like, and then at least two fitness actions may be divided into a group based on the user input. The embodiments of the present disclosure do not limit the implementations of grouping of the fitness actions.

In this case, the action quality of a fitness action group may be evaluated. For example, a value of one or more action evaluation metrics corresponding to the fitness action group is determined. In some examples, the motion data corresponding to at least two fitness actions that are divided into a group is taken as a motion data segment. In this case, the motion data segment includes the motion data corresponding to at least two fitness actions belonging to the same group. In some other examples, the motion data corresponding to at least two fitness actions that are divided into a group belongs to different motion data segments. For instance, a motion data segment contains the motion data of one fitness action. In this case, based on the value of the one or more action evaluation metrics corresponding to each of the at least two motion data segments, or corresponding metric feature data associated with each of the at least two motion data segments, the value of the one or more action evaluation metrics corresponding to at least two fitness actions included in the group may be obtained. In an example, the metric feature data corresponding to the fitness action group may be determined based on the metric feature data for each of the fitness actions in a fitness action group, and then, the value of the one or more action evaluation metrics corresponding to the fitness action group is determined based on the calculated metric feature data. In another example, the value of the one or more action evaluation metrics for each fitness action in the fitness action group may be determined, and then, the value of the one or more action evaluation metrics corresponding to the fitness action group is obtained based on the value of the one or more action evaluation metrics for each fitness action.

It should be understood that, the above-mentioned embodiments are described by taking the fitness action group including two or more fitness actions as an example. In some other embodiments, the fitness action group may include a single fitness action, and the quality evaluation of the fitness action group is assessed similarly to the determination of the value of the at least one action evaluation metric for the single fitness action, which will not be repeated herein.

Optionally, an overall quality evaluation of the current fitness or workout session of the user may further be performed. For instance, the action quality of all fitness actions of the user during the current workout or fitness session may be determined based on a plurality of motion data segments. e.g., the value of at least one action evaluation metric corresponding to the current fitness session is determined.

In the foregoing embodiments, the value of the action evaluation metric may be determined in various manners based on the metric feature data. In some embodiments, mapping processing may be performed on the metric feature data to obtain the value of the action evaluation metric. In an example, the metric feature data may be mapped based on a predetermined mapping function or mapping rule to obtain the value of the action evaluation metric. In another example, at least one of a mapping function, a mapping parameter, or a mapping rule is determined based on at least one of the data related to the current fitness session, user attribute data, or historical motion data of the user. The data related to the current fitness session may include, for example, one or more of user input, the above-mentioned motion data, exercise time duration, exercise starting time point or ending time point, physiological data collected during the workout process (i.e., the fitness session) or the like. The user attribute data for example, may include one or more of grouping of the user, age, gender, body mass index (BMI), fitness goal, fitness habit or the like.

In an example, a mapping function between the metric feature data associated with an action evaluation metric and the value (e.g., score) of the action evaluation metric may be obtained in advance in the following manner.

First, during the fitness sessions of one or more users, at least one sensor is utilized to collect the motion data of the user to obtain a plurality pieces of motion data. Meanwhile, a camera is utilized to shoot videos of the fitness sessions to obtain a plurality of fitness videos.

Next, for the plurality of pieces of collected motion data, the metric feature data associated with an action evaluation metric corresponding to each piece of motion data is calculated, and professional fitness coaches and/or ordinary users are invited to score the plurality of fitness videos with respect to the action evaluation metric, so as to obtain the metric feature data and subjective scores corresponding to the same action evaluation metric for the same one or more fitness actions respectively. In this case, a correspondence between the metric feature data and the subjective score of the action evaluation metric is obtained.

Subsequently, function fitting is performed based on the correspondence to obtain a mapping function between the metric feature data associated with the action evaluation metric and the value of the action evaluation metric.

In some embodiments, prior knowledge may be further taken into account in determining the mapping function or rule. In an example, the mapping function obtained in the above example may be adjusted based on the prior knowledge, or function fitting may be performed based on the prior knowledge. e.g., fine tuning of key points may be performed based on the prior knowledge, and re-fitting is performed after some key points are raised or lowered. In another example, a score range is set to [0, 100], and a portion exceeding the score range is truncated to the boundary value. In another example, a piece-wise function is utilized as the mapping function, or a piece-wise function is utilized for fine-tuning, etc.

In some embodiments, feedback from the user, a coach, or other subject may be received. For example, feedback is received after the action quality evaluation result is output. Alternatively, subjective evaluation input from the user, the coach, or other subject may be received. In this case, the current mapping function or rule may be adjusted for iterative optimization based on at least one of the received feedback and subjective evaluation input.

After obtaining the mapping relationship between the metric feature data associated with a target action evaluation metric (e.g., any action evaluation metric) and the value of the target action evaluation metric based on the aforementioned method, the value of the target action evaluation metric may be determined based on the calculated metric feature data associated with the target action evaluation metric.

It should be noted that, the foregoing description of determining the mapping relationship between the metric feature data and the value of the action evaluation metric is merely illustrative. In practical applications, the mapping relationship between the metric feature data and the value of the target action evaluation metric may be obtained based on other fitting mechanisms, and the present disclosure is not limited thereto.

It should be understood that, in the embodiments of the present disclosure, the value of the action evaluation metric may be a score or an indicator, such as "high", "medium", "low", or "excellent", "good", "fair", "poor", or the like. The present application does not impose any limitation on the specific implementations of the value of the action evaluation metric.

In the embodiments of the present disclosure, by performing the data reconstruction processing, a same algorithm or model can be used to determine the values of the action evaluation metrics without considering the specific exercise type to which the fitness actions belong, which has a high degree of universality, not only reduce the complexity of the overall procedure, but also avoid the influence of the accuracy of the algorithm for exercise type recognition on the action quality evaluation result, thereby contributing to improvement of the accuracy of the action quality evaluation result.

At 203, an action quality evaluation result of the user is determined according to the value of the at least one action evaluation metric.

As mentioned above, for a certain action evaluation metric, at least one of the following may be obtained: one or more values of the action evaluation metric for one or more fitness actions, one or more values of the action evaluation metric for a fitness action group, or one or more values of the action evaluation metric for the entire fitness session.

In some embodiments, the action quality evaluation result may include at least one of: a value of each of the at least one action evaluation metric, a statistical result of the value of the at least one action evaluation metric, evaluative description of each of the at least one action evaluation metric, or a suggestion for the user to improve the actions. In an example, a value of the at least one action evaluation metric corresponding to the fitness session may be obtained based on the value of the at least one action evaluation metric obtained at 202.

In another example, the values of a certain action evaluation metric obtained at 202 may be statistically processed, so as to obtain an average value of the action evaluation metric corresponding to the fitness actions performed by the user during the fitness session, or obtain a maximum or minimum value of the action evaluation metric and a position of the corresponding fitness action, or obtain a number of fitness actions, a proportion of fitness action, or positions of fitness actions whose values of the action evaluation metric reach a preset threshold, or obtain a number of fitness actions, a proportion of fitness actions, or positions of fitness actions whose values of the action evaluation metric do not reach the preset threshold, etc.

In another example, personalized feedback, such as at least one of an evaluative description (e.g., qualitative summaries of performance) or an action improvement suggestion, for one or more fitness actions performed by the user may be obtained based on the value of the at least one action evaluation metric. The action improvement suggestion may be actionable recommendations to improve action quality. The evaluative descriptions may be, for example, the user performs one or more fitness actions with poor stability, or the user has relatively good strength control in performing one or more fitness actions, etc. In some other examples, the value of the action evaluation metric is compared to a preset threshold to determine whether a desired fitness effect is achieved or whether there is a risk of injury. In an example, if the intensity variability of a plurality of fitness actions indicate that an attenuation degree of the plurality of fitness actions is less than a preset threshold, such as 10%, it is determined that the execution of the plurality of fitness actions does not achieve the desired fitness effect. In another example, if the intensity variability of the plurality of fitness actions is higher than a preset threshold, such as 50%, it is determined that the user has a relatively high risk of injury. In this case, optionally, at least one of the evaluative description and action improvement suggestion may be output during the fitness session of the user, so as to facilitate the user to make adjustment while performing subsequent fitness actions.

In some embodiments, the action quality evaluation result may be output. In some examples, the action quality evaluation result may be output via an intermediate device, a wearable device, or other devices. In an example, the action quality evaluation result may be displayed on a visualization interface, such as on the screen of the wearable device or the intermediate device. In another example, the action quality evaluation result may be output via an audio output device. The specific implementations of the output device and the specific output manners are not limited herein.

In some embodiments, exercise type data input by the user may be received. In this case, while outputting the action quality evaluation result, the corresponding exercise type data may also be output.

In some embodiments, the value of the at least one action evaluation metric may be processed based on an LLM (Large Language Model) to obtain the action quality evaluation result. In this case, the action quality evaluation result may include comprehensive information about the action quality of the user in terms of the at least one action evaluation metric.

In some examples, the value of the at least one action evaluation metric obtained as described above may be converted into a structured description conforming to a preset data format, such as a json schema format. Then, the converted description in the json schema format is input into the LLM, and the action quality evaluation result output by the LLM is obtained.

In some examples, the action quality evaluation result output by the LLM is similar to an action quality evaluation of the fitness actions performed by the user during the workout process made by a professional fitness coach. For example, the action quality evaluation result may be a structured report such as: Your overall performance in the workout session is excellent and has an overall score of 85 (e.g., Overall Performance: 85, Excellent).For the barbell bench press, your overall action consistency score is 80, but the 6th 7th, and 9th actions are poorly performed with an action consistency score lower than the standard (e.g., Action Consistency: 80, Note: the actions #6, #7 and #9 are poorly performed with a score fell below the standard); your overall action stability score is 90 with only the 7th action being slightly worse (e.g., Action Stability: 90, Note: action #7 is slightly worse); your action continuity score is 70, and the 7th and 9th actions need to be improved (e.g., Action Continuity: 70, Note: actions #7 and #9 need to be improved); your action variability is well controlled and has a score of 90, but there is a slight decline in performing the 4th action (e.g., Action Variability: 90, Note: there is a slight decline in performing the action #7). For the barbell squat, your action consistency and action stability are well performed, both of which have a score above 90 (e.g., Action Consistency: 92; Action Stability: 94). For the bench press, attention should be given to improve the action continuity and continue to maintain good action stability.

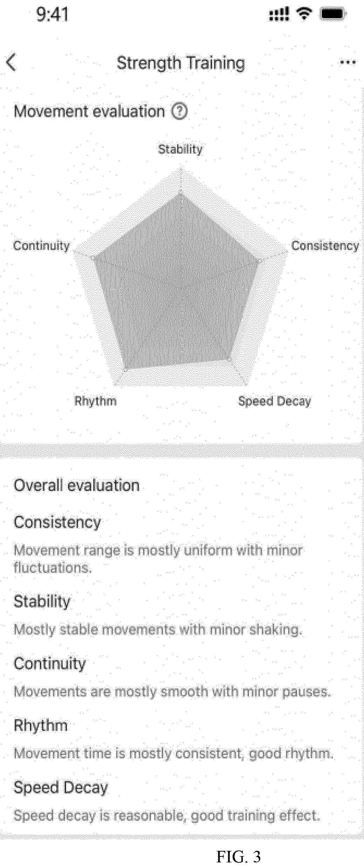

It should be understood that the foregoing description of the action quality evaluation result output by the LLM is merely illustrative, and is intended to enable those skilled in the art to better understand the technical solutions of the embodiments of the present disclosure. In practical applications, the action quality evaluation result output by the LLM may be realized in other forms, such as in the form of a table or in the form of a radar chart as shown in FIG. 3, which is not limited in the present disclosure.

FIG. 3 illustrates an exemplary user interface for displaying the action quality evaluation result in a scenario of strength training. The interface includes two sections: movement evaluation and overall evaluation, and is designed to provide both granular and holistic feedback to the user. In the movement evaluation section, the evaluation results of stability, continuity, rhythm (i.e., completion time consistency), speed decay (i.e., variability) and consistency (i.e., amplitude consistency) are presented with a radar chart. In the overall evaluation section, the evaluation results of the respective action evaluation metrics are described in a more general and summarized manner, which may be generated with the LLM.

In the scheme for action quality evaluation provided in the embodiments of the present disclosure, the action quality evaluation result determined based on the value of one or more action evaluation metrics is utilized to characterize the action quality of the user, which is easier for the user to understand, and thus satisfy the user's needs.

The determination of the metric feature data or the value of each action evaluation metric for one or more fitness actions will be described in detail below with specific examples.

The action consistency is used for evaluating at least one of amplitude consistency or completion time consistency of a plurality of fitness actions performed by the user, where the plurality of fitness actions may be multiple fitness actions included in a single motion data segment, multiple fitness actions included in a fitness action group, or multiple fitness actions included in the entire fitness session, etc.

In some implementations, the amplitude consistency of the plurality of fitness actions may be determined in the following manner: reconstructed displacement data for each fitness action, such as virtual principal axis displacement data (i.e., the virtual principal axis data of displacement), is determined based on the reconstructed data for the each fitness action, and then the metric value (i.e., value) of the amplitude consistency of the plurality of fitness actions is determined based on the reconstructed displacement data for each of the plurality of fitness actions.

In some examples, a statistical operation is performed on the reconstructed displacement data corresponding to a fitness action to obtain statistical data, and the metric feature data corresponding to the fitness action may be obtained based on the statistical data.

For instance, at least one of the peak region data (e.g., the first few largest values) or the trough region data (e.g., the first few smallest values) in the virtual principal axis displacement data corresponding to a fitness action may be determined, and at least one of the peak region data or the trough region data, such as 90% of the displacement peak, an average of the data above 90% of the displacement peak or the like, is taken as the metric feature data. In this case, a lower value of the metric feature data indicates a higher action amplitude consistency during the execution of the plurality of fitness actions by the user and a higher action quality.

For another instance, at least one of the peak region data or the trough region data in the virtual principal axis displacement data for each fitness action may be normalized, for example, by using the peak value (i.e., the maximum value) of the virtual principal axis displacement data corresponding to the fitness action, so as to obtain the metric feature data.

Subsequently, the metric value (i.e., value) of the amplitude consistency of the plurality of fitness actions may be obtained based on the metric feature data for each of the plurality of fitness actions.

In some embodiments, the metric feature data for each of the plurality of fitness actions is normalized to obtain the normalized metric feature data, and then the amplitude consistency of the plurality of fitness actions is obtained based on the normalized metric feature data. For example, a statistical measure (e.g. the maximum value) of the metric feature data associated with the plurality of fitness actions is utilized to normalize the metric feature data for each of the plurality of fitness actions.

In some other embodiments, after obtaining the metric feature data for each of the plurality of fitness actions, the metric feature data for each of the plurality of fitness actions is averaged to obtain the metric feature data for the plurality of fitness actions, and the amplitude consistency of the plurality of fitness actions is obtained based on the metric feature data for the plurality of fitness actions.

In some implementations, the completion time consistency (also referred to as rhythms) of the plurality of fitness actions may be determined in the following manner: an action completion time for each fitness action is determined based on a time duration of the motion data for the each fitness action, and then the completion time consistency of the plurality of fitness actions is determined based on the action completion time for each of the plurality of fitness actions.

In an example, a statistical measure of the action completion times for the plurality of fitness actions, such as the variance, the standard deviation or the like, is taken as the metric feature data associated with the completion time consistency. For instance, a difference between the action completion times corresponding to adjacent fitness actions of the plurality of fitness actions is calculated, and the standard deviation of at least two obtained differences is calculated as the metric feature data associated with the completion time consistency of the plurality of fitness actions.

In this case, a smaller value of the metric feature data indicates a smaller difference in the completion times of the plurality of fitness actions performed by the user, and a higher completion time consistency indicates a higher action quality.

The action stability is used for evaluating a degree of jitter or wobbling of one or more fitness actions performed by the user. The action stability may include at least one of displacement stability or angle stability. The displacement stability may be used for evaluating the jitter degree of the displacements (e.g., high-frequency oscillations) of at least a part of the user's body in one or more directions during the execution of a single fitness action. The angle stability is used for evaluating the wobbling degree or rotation degree of the angles (e.g., angle deviations) of at least a part of the user's body in one or more directions during the execution of the fitness action.

In some embodiments, the virtual secondary axis displacement data (i.e., the virtual secondary axis data of displacement) of a fitness action may be used as the basis for deriving the metric feature data associated with the displacement stability. In some examples, at least one of the peak region data or the trough region data of the virtual secondary axis displacement data is determined, and the metric feature data associated with the displacement stability is determined according to at least one of the peak region data or the trough region data of the virtual secondary axis displacement data. For instance, a ratio of a displacement value corresponding to the second quantile of the virtual secondary axis displacement data to a displacement value corresponding to the third quantile of the virtual secondary axis displacement data is calculated, and the calculated ratio is taken as the metric feature data associated with displacement stability. The second quantile may be set to 90%, or a higher or lower value close to the peak. The third quantile may be 10%, or a higher or lower value close to the trough. In this case, a smaller ratio indicates a smaller jitter amplitude of the displacements of the fitness action performed by the user, which in turn indicates a better displacement stability and a higher action quality.

In some embodiments, the virtual principal axis Euler angle data (i.e., the virtual principal axis data of Euler angle) of a fitness action is taken as the basis for deriving the metric feature data associated with angle stability. In some examples, at least one of the peak region data or the trough region data of the virtual principal axis Euler angle data is determined, and the metric feature data associated with the angle stability is determined according to at least one of the peak region data or the trough region data of the virtual principal axis Euler angle data. For instance, after cosine conversion is performed on the virtual principal axis Euler angle data, a ratio of the Euler angles corresponding to its fourth quantile and its fifth quantile is calculated, and the ratio is taken as the metric feature data associated with angle stability. The fourth quantile is set to 85%, or a higher or lower value close to the peak. The fifth quantile is set to 15%, or a higher or lower value close to the trough. In this case, a smaller ratio indicates a smaller wobbling or rotation amplitude (e.g., a smaller angular deviation) of the fitness action performed by the user, which in turn indicates a better action stability and a higher action quality.

In some embodiments, fused metric feature data is further obtained based on the metric feature data associated with the angle stability and the metric feature data associated with the displacement stability. For example, the metric feature data associated with the angle stability and the metric feature data associated with the displacement stability are summed with weights to obtain the fused metric feature data. In this case, the metric feature data associated with the action stability may include at least one of the metric feature data associated with the angle stability, the metric feature data associated with the displacement stability, or the fused metric feature data.

In some embodiments, after obtaining the metric feature data associated with the action stability for each of the plurality of fitness actions, statistical processing or aggregation is performed on the metric feature data associated with the action stability for each of the plurality of fitness actions, such as weighted averaging, averaging, or normalization or the like, to obtain the metric feature data associated with the action stability for the plurality of fitness actions, and then the metric value (i.e., value) of the action stability for the plurality of fitness actions is obtained.

In some other embodiments, the metric value of the action stability for each fitness action is obtained based on the metric feature data associated with the action stability for the each fitness action, and the metric value of the action stability for the plurality of fitness actions is obtained based on the metric values of the action stability for each of the plurality of fitness actions, which is not limited in the present disclosure.

The action continuity is used for evaluating the continuity degree of one or more fitness actions performed by the user.

In some embodiments, the metric feature data associated with the action continuity corresponding to a fitness action is obtained based on the virtual principal axis data of acceleration and/or velocity associated with the fitness action. In some examples, a ratio of a time duration, during which the value of the virtual principal axis velocity data of the fitness action is less than or equal to a preset velocity threshold, to a total time duration of the fitness action is calculated. Alternatively, a ratio of a time duration, during which the value of the virtual principal axis acceleration data is less than or equal to a preset acceleration threshold, to the total time duration of the fitness action is calculated. The obtained ratio may be taken as the aforementioned metric feature data associated with the action continuity. In this case, a smaller ratio indicates a fewer sudden or unnecessary pauses during the fitness action performed by the user, a higher degree of continuity of the fitness action, and a higher action quality.

In some embodiments, after obtaining the metric feature data associated with the action continuity for each of the plurality of fitness actions, statistical processing, such as weighted averaging, averaging, or normalization or the like, may be performed on the metric feature data associated with the action continuity for each of the plurality of fitness actions, to obtain the metric feature data associated with the action continuity for the plurality of fitness actions, and then the metric value (i.e., value) of the action continuity for the plurality of fitness actions is obtained.

In some other embodiments, the metric value of the action continuity for each fitness action is obtained based on the metric feature data associated with the action continuity for the each fitness action, and the metric value of the action continuity for the plurality of fitness actions is obtained based on the metric values of the action continuity for each of the plurality of fitness actions, which is not limited in the present disclosure.

The action variability (such as speed decay) is used for evaluating an intensity change of a plurality of fitness actions performed by the user. The action variability indicates whether the user reaches a certain degree of exhaustion and/or whether there is a risk of injury during the execution of the plurality of fitness actions.

In the strength training, a training effect of the plurality of fitness actions is usually measured by determining whether the trainer reaches a certain degree of exhaustion. Therefore, the training effect of the trainer is measured by evaluating the attenuation of velocity or acceleration during the execution of the plurality of fitness actions.

In some embodiments, the metric feature data associated with the action variability corresponding to a fitness action is obtained based on the virtual principal axis data of velocity and/or acceleration. In some examples, statistical processing, such as averaging, is performed on the virtual principal axis data of velocity and/or acceleration corresponding to the fitness action, to obtain a statistical measure of the virtual principal axis data of velocity and/or acceleration corresponding to the fitness action. Subsequently, a statistical measure of the virtual principal axis data of velocity and/or acceleration of the first M fitness actions is calculated, and a statistical measure of the virtual principal axis data of velocity and/or acceleration of the last N fitness actions of the plurality of fitness actions is calculated, so as obtain the metric feature data associated with the action variability corresponding to the pluraitly of fitness actions. Both M and N are integers, where M≥1 and N≥1. At least one of M or N may be preset or determined according to the number of the plurality of fitness actions. For instance, M and/or N are set to 20% of the number of the plurality of fitness actions. Alternatively, M and/or N are set to a percentage that is higher or lower than 20% of the number of the plurality of fitness actions.

In some examples, a difference or a proportion of change between the statistical measure of the virtual principal axis data of velocity and/or acceleration of the first M fitness actions and the statistical measure of the virtual principal axis data of velocity and/or acceleration of the last N fitness actions is calculated, and taken as the metric feature data associated with the action variability for the plurality of fitness actions.

In some other examples, after obtaining the metric feature data based on the virtual principal axis data of acceleration (referred to as acceleration-based metric feature data) and the metric feature data based on the virtual principal axis data of velocity (referred to as velocity-based metric feature data), the acceleration-based metric feature data and the velocity-based metric feature data are fused, for example, by weighted summation or weighted averaging, to obtain the fused metric feature data. The fused metric feature data is taken as the metric feature data associated with the action variability for the plurality of fitness actions.

The force control is used for evaluating the strength exertion condition of one or more fitness actions performed by the user.

The force control is used for assessing whether the user completes a fitness action by relying non-essentially on inertia or elasticity, or whether there is an unnecessary sudden exertion of force during the execution of an fitness action, which may be determined by evaluating whether there is a sharp change in the force applied by the user while performing the fitness action.

In some embodiments, the metric feature data associated with the force control corresponding to a fitness action is obtained based on the virtual secondary axis acceleration data (i.e., the virtual secondary axis data of acceleration) corresponding to the fitness action. In some examples, one or more types of statistical operations, such as averaging and normalization, are performed on the virtual secondary axis acceleration data corresponding to the fitness action to obtain the metric feature data associated with the force control corresponding to the fitness action. It can be understood that, a smaller value of the metric feature data indicates that the user relies less on inertia or elasticity to completes the action while performing the fitness action. If there is fewer drastic changes in the force applied by the user while performing the fitness action, it indicates a better force control and a higher action quality.

In some embodiments, after obtaining the metric feature data associated with the force control for each of the plurality of fitness actions, statistical processing, such as weighted averaging, averaging, or normalization or the like, is performed on the metric feature data associated with the force control corresponding to each of the multiple fitness actions, to obtain the metric feature data associated with the force control for the plurality of fitness actions. Subsequently, the metric value (i.e., value) of the force control for the plurality of fitness actions is obtained.

In some other embodiments, the metric value (i.e., value) of the force control for each fitness action is obtained based on the metric feature data associated with the force control for the each fitness action, and the metric value of the force control for the plurality of fitness actions is obtained based on the metric values of the force control for each of the plurality of fitness actions, which is not limited in the present disclosure.

The action normality is used for evaluating a normality or standardization degree of one or more fitness actions performed by the user.

In some embodiments, the metric feature data associated with the action normality corresponding to a fitness action is obtained based on the virtual principal axis acceleration data of the fitness action. In some examples, the virtual principal axis acceleration data of the fitness action is compared with preset virtual principal axis acceleration reference data (e.g., the virtual principal axis acceleration data corresponding to a reference action). e.g., a similarity between the virtual principle axis acceleration data and the virtual principal axis acceleration reference data is calculated using an algorithm such as DTW (dynamic time warping) or the like, and the similarity is taken as the metric feature data associated with the action normality. The advantage of the DTW algorithm is that it does not require the two pieces of data to have the same length, and thus can avoid the influence of a difference in action periods on the similarity. In some other examples, a similarity or difference between the virtual principal axis acceleration data of a certain fitness action and the virtual principal axis acceleration data of at least one adjacent fitness action may be determined, and the metric feature data associated with the action normality corresponding to the fitness action is obtained according to the similarity or difference.

In this case, a larger value of the metric feature data indicates a higher similarity between the fitness action performed by the user and the reference action, a better action normality, and a higher action quality.

In some embodiments, after obtaining the metric feature data associated with the action normality for each of the plurality of fitness actions, statistical processing, such as weighted averaging, averaging, or normalization or the like, is performed on the metric feature data associated with the action normality corresponding to each of the multiple fitness actions, to obtain the metric feature data associated with the action normality for the plurality of fitness actions, and then the metric value of the action normality for the plurality of fitness actions is obtained.

In some other embodiments, the metric value of the action normality for each fitness action is obtained based on the metric feature data associated with the action normality for the each fitness action, and the metric value of the action normality for the plurality of fitness actions is obtained based on the metric values of the action normality for each of the plurality of fitness actions, which is not limited in the present disclosure.

The action balance is used for evaluating the left-right balance degree of one or more fitness actions performed by the user. This metric is applicable to symmetric fitness actions performed by the user. In this case, the above-mentioned motion data is obtained from the sensor data collected by a set of sensors located at symmetric or nearly symmetric body parts of the user, such as the left and right wrists, left and right arms, left and right chests, left and right torsos, left and right ankles, left and right legs, left and right ears, left and right eyes of the user, etc.

In some embodiments, the metric value of the action balance is obtained by determining a similarity or difference between the motion data corresponding to a fitness action that is obtained from a sensor on the left side and that obtained from a sensor on the right side. In some examples, the metric feature data associated with the action balance is obtained based on the virtual principal axis velocity data (i.e., the virtual principal axis data of velocity). For instance, a difference between the virtual principal axis velocity data based on the left-side sensor and the virtual principal axis velocity data based on the right-side sensor corresponding to a same fitness action is determined. The metric feature data associated with the action balance for the fitness action is obtained based on an average of the differences between the virtual principal axis velocity data based on the left-side sensor and the virtual principal axis velocity data based on the right-side sensor. Alternatively, the metric feature data associated with the action balance for the fitness action is determined based on a difference between the average of the virtual principal axis velocity data based on the left-side sensor and the average of the virtual principal axis velocity data based on the right-side sensor. Alternatively, the metric feature data associated with the action balance for the fitness action is determined based on a ratio of change in the virtual principal axis velocity data derived from the left-side sensor and the right-side sensor, or a ratio of change in the average of the virtual principal axis velocity data derived from the left-side sensor and the right-side sensor.

It can be understood that, a smaller value of the metric feature data indicates a better left-right balance of the user while performing the fitness action, and a higher action quality.

In some embodiments, after obtaining the metric feature data associated with the action balance for each of the plurality of fitness actions, statistical processing, such as weighted averaging, averaging, or normalization or the like, is performed on the metric feature data associated with the action balance corresponding to each of the plurality of fitness actions, to obtain the metric feature data associated with the action balance corresponding to the plurality of fitness actions, and then the metric value of the action balance for the plurality of fitness actions is obtained.

In some other embodiments, the metric value of the action balance for each fitness action is obtained based on the metric feature data associated with the action balance for the each fitness action, and the metric value of the action balance for the plurality of fitness actions is obtained based on the metric values of the action balance for each of the plurality of fitness actions, such as by averaging, weighted averaging or the like, which is not limited in the present disclosure.

The foregoing is described by taking the virtual principal axis velocity data as an example. In some embodiments, the metric feature data associated with the action balance for a fitness action may be determined based on at least one of the virtual principal axis acceleration data, the virtual principal axis displacement data, or the virtual principal axis Euler angle data, which will not be repeated herein.

Optionally, during extraction of the metric feature data associated with each of the above-mentioned action evaluation metrics, related values, statistical values, or normalized values may be used, which can effectively mitigate the impacts arising from the differences between different types of fitness actions and/or the differences between different individuals in performing the same fitness actions, thereby improving the accuracy and applicability of the action quality evaluation result.

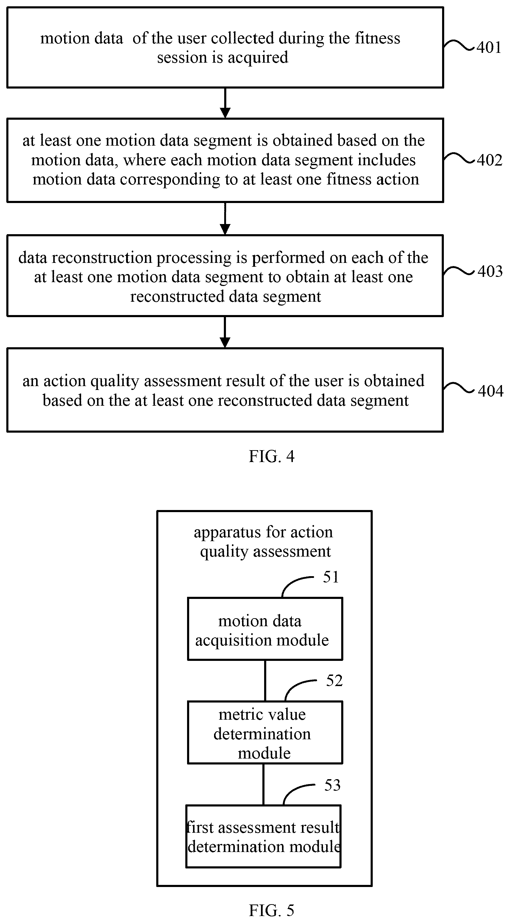

FIG. 4 is another flowchart of the method for action quality evaluation in some exemplary embodiments of the present disclosure. As shown in FIG. 4, the method in the exemplary embodiments may include 401 to 403.

At 401, motion data of the user collected during the fitness session is acquired.

The motion data may include at least one of acceleration data, velocity data, displacement data, or Euler angle data.

At 402, at least one motion data segment is obtained based on the motion data, where each motion data segment includes motion data corresponding to at least one fitness action.

In some embodiments, the motion data is segmented to obtain multiple motion data segments, where each motion data segment may correspond to one or more fitness actions. For example, the motion data of two fitness actions performed by the user is segmented into a motion data segment corresponding to fitness action #1 and a motion data segment corresponding to fitness action #2.

At 403, data reconstruction processing is performed on each of the at least one motion data segment to obtain at least one reconstructed data segment.

In some embodiments, data reconstruction processing is performed on each of the multiple motion data segments in the following manner: for each motion data segment, the data in the motion data segment is decomposed using a singular value decomposition (SVD) algorithm to obtain multi-axis processed data. The SVD algorithm is used to effectively separate the primary and secondary signal responses from the motion data segment. For example, the SVD is performed on a three-axis motion signal $A$ (t×3) to obtain three matrices: $U$ (t×3), $\Sigma$ (3), and $V$ (3×3). Then, data reconstruction processing is performed on the obtained multi-axis processed data to obtain the reconstructed data segment. For instance, continuing with the above example, the first two features of $\Sigma$ (i.e., the signals (i.e., values) of the first two orthogonal axes of the matrix $\Sigma$) are reconstructed to obtain a virtual principal axis signal, which represents dominant motion patterns, and the last feature of $\Sigma$ is reconstructed to obtain a virtual secondary axis signal, which represents auxiliary motion patterns such as noises. It is assumed that the virtual principal axis signal is represented by $Axis_m$, and it may be obtained by the following formula: $Axis_m = U_2 \Sigma_2 V_2^t$. It is assumed that the virtual auxiliary-axis signal is represented by $Axis_i$, and it may be obtained by the following formula: $Axis_i = U_1 \Sigma_1 V_1^t$. Optionally, the data reconstruction processing may be performed in other ways, which is not limited herein.

In some embodiments, the obtained reconstructed data segment may include at least one of the virtual principal axis data or the virtual secondary axis data. In practical applications, the data reconstruction is performed on the data of at least two axes in the multi-axis processed data obtained from the data decomposition, to obtain the virtual principal axis data, where the data of the at least two axes exhibits strong signal responses. The data reconstruction is performed on the data of at least one axis in the multi-axis processed data to obtain virtual secondary axis data, where the data of the at least one axis exhibits weak signal responses. In this case, the obtained virtual principal axis data is used to characterize the signal responses to the primary motion, and the virtual secondary axis data is used to characterize the signal responses to sources other than the primary motion.

At 404, an action quality evaluation result of the user is obtained based on the at least one reconstructed data segment.

In some embodiments, obtaining the action quality evaluation result of the user based on the at least one reconstructed data segment includes: determining a value of at least one action evaluation metric for each of the at least one motion data segment according to the at least one reconstructed data segment; and determining the action quality evaluation result of the user according to the value of the at least one action evaluation metric for each of the at least one motion data segment.

It can be seen from the above descriptions of the determination of the metric feature data associated with each of the action evaluation metrics that, the value of the at least one action evaluation metric for one or more fitness actions performed by the user may be determined based on the virtual principal axis data in the reconstructed data segment, where the at least one action evaluation metric includes at least one of action consistency, action stability, action continuity, action variability, action normality, or action balance. Furthermore, the value of the at least one action evaluation metric for one or more fitness actions performed by the user may be determined based on the virtual secondary axis data in the reconstructed data segment, where the at least one action evaluation metric includes at least one of action stability or force control.

For the specific implementations of the method for action quality evaluation, reference may be made to any of the foregoing embodiments, which will not be repeated herein.

In the method for action quality evaluation provided by the embodiments of the present disclosure, after the motion data of the user collected during the fitness process is acquired, the motion data is segmented to obtain at least one motion data segment. Subsequently, the data reconstruction processing is performed on each motion data segment to obtain at least one reconstructed data segment. Finally, the action quality evaluation result of the user is obtained based on the at least one reconstructed data segment. In the present solution, by performing reconstruction processing on the motion data segment to obtain the reconstructed data segment, the influence of different exercise types can be mitigated or eliminated. Therefore, the determination of the value of each action evaluation metric is independent of the exercise type. That is to say, for different types of fitness actions, the action quality evaluation result may be determined based on the same algorithm or model, which demonstrates a high level of universality.

FIG. 5 is a schematic structural diagram of an apparatus for action quality evaluation shown in an exemplary embodiment of the present disclosure. As shown in FIG. 5, the action quality evaluation device may include the following modules.

A motion data acquisition module 51, configured to acquire motion data of a user collected during a fitness session.

A metric value determination module 52, configured to determine a value of at least one action evaluation metric of the user based on the motion data.

A first assessment result determination module 53, configured to determine an action quality evaluation result of the user according to the value of the at least one action evaluation metric.

The at least one action evaluation metric includes at least one of:

action consistency, for evaluating at least one of amplitude consistency or completion time consistency of multiple fitness actions performed by the user;

action stability, for evaluating a degree of jitter or wobbling of one or more fitness actions performed by the user;

action continuity, for evaluating a continuity degree of one or more fitness actions performed by the user;

action variability, for evaluating an intensity variability of multiple fitness actions performed by the user; or

force control, for evaluating force exertion condition during one or more fitness actions performed by the user.

Optionally, when the metric value determination module 52 is configured to determine the values of at least one action evaluation metric of the user based on the motion data, it includes: determining the metric feature data for each of at least one fitness action performed by the user based on the motion data, and obtaining the value of at least one action evaluation metric for the user according to the metric feature data for each of the at least one fitness action.

Optionally, the determination of the metric feature data for each of the at least one fitness action does not depend on the exercise type of the each fitness action.

Optionally, the determination of the metric feature data for each of the at least one fitness action includes performing data reconstruction processing on the motion data, where the data reconstruction processing is used to mitigate or eliminate the influence of the exercise type.

Optionally, the metric feature data for each of the at least one fitness action is obtained by performing singular value decomposition processing on the motion data.

Optionally, when the metric value determination module 52 is configured to determine the value of at least one action evaluation metric of the user based on the motion data, it includes: obtaining at least one motion data segment according to the motion data, each motion data segment including motion data corresponding to at least one fitness action, performing data reconstruction processing on each of the at least one motion data segment to obtain at least one reconstructed data segment, and obtaining the value of the at least one action evaluation metric of the user according to the at least one reconstructed data segment.

Optionally, the reconstructed data segment includes at least one of virtual principal axis data or virtual secondary axis data, where the virtual principal axis data is used to characterize the signal response to the primary motion, and the virtual secondary axis data is used to characterize the signal response to sources other than the primary motion.

Optionally, the action quality evaluation result of the user includes at least one of: a statistical result of the value of the at least one action evaluation metric of the user; evaluative description of the at least one action evaluation metric of the user; or an action improvement suggestion for the user.

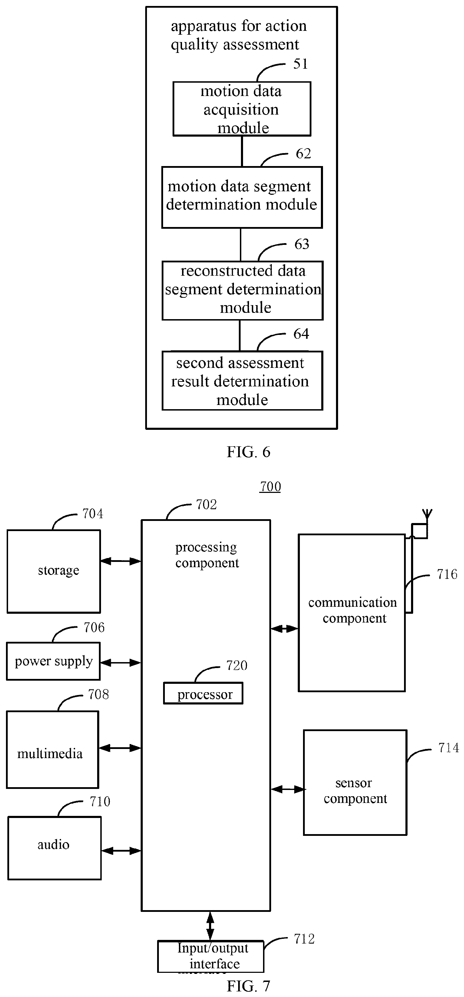

FIG. 6 is a schematic structural diagram of another apparatus for action quality evaluation in an exemplary embodiment of the present disclosure. As shown in FIG. 6, the apparatus for action quality evaluation may include the following modules.

A motion data acquisition module 51 is configured to acquire motion data of a user collected during a fitness session.

A motion data segment determination module 62 is configured to obtain at least one motion data segment based on the motion data, where each motion data segment includes motion data corresponding to at least one fitness action.

A reconstructed data segment determination module 63 is configured to perform data reconstruction processing on each of the at least one motion data segment to obtain at least one reconstructed data segment.

A second assessment result determination module 64 is configured to obtain the action quality evaluation result of the user based on the at least one reconstructed data segment.

Optionally, the data reconstruction processing is used to mitigate or eliminate the influence of the exercise type.

Optionally, the data reconstruction processing includes singular value decomposition processing.

Optionally, the reconstructed data segment includes at least one of virtual principal axis data or virtual secondary axis data, where the virtual principal axis data is used to characterize the signal response to the primary motion, and the virtual secondary axis data is used to characterize the signal response to sources other than the primary motion.

Optionally, when the second assessment result determination module 64 is configured to obtain the action quality evaluation result of the user based on the at least one reconstructed data segment, it includes: determining the value of at least one action evaluation metric for one or more fitness actions performed by the user according to the virtual principal axis data in the reconstructed data segment, where the at least one action evaluation metric includes at least one of action consistency, action stability, action continuity, action variability, or action normality.

Optionally, when the second assessment result determination module 64 is configured to obtain the action quality evaluation result of the user based on the at least one reconstructed data segment, the second assessment result determination module 64 is configured to determine the value of at least one action evaluation metric for one or more fitness actions performed by the user according to the virtual secondary axis data in the reconstructed data segment, where the at least one action evaluation metric includes at least one of action stability or force control.

The implementation details of the functions and roles of each module in the above-mentioned apparatuses are elaborated in the corresponding implementations of the methods described above, which will not be repeated herein.

The embodiments of the apparatuses basically correspond to the embodiments of the methods, and reference can be made to the descriptions of the embodiments of the methods.

In some embodiments, the present disclosure further provides a computer-readable storage medium having a computer program stored thereon. When the program is executed by a processor, any of the above-mentioned methods are implemented.

In some embodiments, the present disclosure further provides a computer program product, including a computer program or instructions. When the computer program or instructions is executed by a processor, the method described in any of the above-mentioned embodiments is implemented.

As shown in FIG. 7, FIG. 7 is a structural diagram of an electronic device in an exemplary embodiment of the present disclosure. The device 700 may be a smart phone or mobile phone, a tablet, a laptop, a desktop computer, a wearable device (e.g., a watch, glasses, gloves, headgear (e.g., a hat, a helmet, a virtual reality headset, an augmented reality headset, a head-mounted device (HMD), a headband), a pendant, an armband, a leg ring, shoes, a vest), a server, etc.

Referring to FIG. 7, the device 700 may include one or more of the following components: a processing component 702, a memory 704, a power supply component 706, a multimedia component 708, an audio component 710, an input/output (I/O) interface 712, a sensor component 714, and a communication component 716.

The processing component 702 generally controls the overall operation of the device 700, such as operations associated with display, phone calls, data communication, camera operations, and recording operations. The processing component 702 may include one or more processors 720 to execute instructions to complete all or part of the steps of the above-mentioned method. In addition, the processing component 702 may include one or more modules to facilitate the interaction between the processing component 702 and other components. For example, the processing component 702 may include a multimedia module to facilitate the interaction between the multimedia component 708 and the processing component 702.

The memory 704 is configured to store various types of data to support the operation at the device 700. Examples of such data include instructions for any application or method operating on the device 700, contact data, phone book data, messages, pictures, videos, etc. The memory 704 may be implemented by any type of volatile or non-volatile storage device or a combination thereof, such as static random-access memory (SRAM), electrically erasable programmable read-only memory (EEPROM), erasable programmable read-only memory (EPROM), programmable read-only memory (PROM), read-only memory (ROM), magnetic memory, flash memory, a magnetic disk, or an optical disk.

The power supply component 706 provides power to various components of the device 700. The power supply component 706 may include a power management system, one or more power supplies, and other components associated with generating, managing, and distributing power for the device 700.

The multimedia component 708 includes a screen that provides an output interface between the device 700 and the user. In some embodiments, the screen may include a liquid crystal display (LCD) and a touch panel (TP). If the screen includes a touch panel, the screen may be implemented as a touch screen to receive input signals from the user. The touch panel includes one or more touch sensors to sense touches, swipes, and gestures on the touch panel. The touch sensors may sense not only the boundaries of touch or swipe actions, but also the duration and pressure associated with the touch or swipe operations. In some embodiments, the multimedia component 708 includes a front camera and/or a rear camera. When the device 700 is in an operating mode, such as a shooting mode or a video mode, the front camera and/or the rear camera may receive external multimedia data. Each of the front camera and the rear camera may be a fixed optical lens system or have a focal length and optical zoom capabilities.

The audio component 710 is configured to output and/or input audio signals. For example, the audio component 710 includes a microphone (MIC). When the device 700 is in an operating mode, such as a call mode, a recording mode, and a voice recognition mode, the microphone is configured to receive external audio signals. The received audio signals may be further stored in the memory 704 or sent via the communication component 715. In some embodiments, the audio component 710 further includes a speaker for outputting audio signals.

The I/O interface 712 provides an interface between the processing component 702 and peripheral interface modules, which may be a keyboard, a click wheel, buttons, etc. These buttons may include, but are not limited to, a home button, a volume button, a start button, and a lock button.

The sensor component 714 includes one or more sensors for providing various aspects of state assessment for the device 700. For example, the sensor component 714 may detect the on/off state of the device 700, the relative positioning of components, such as the display and the keypad of the device 700. The sensor component 714 may also detect the change in position of the device 700 or a component in the device 700, the presence or absence of user contact with the device 700, the orientation or acceleration/deceleration of the device 700, and the change in temperature of the device 700. The sensor component 714 may include a proximity sensor, configured to detect the presence of nearby objects without any physical contact. The sensor component 714 may further include a light sensor, such as a CMOS or CCD image sensor, for use in imaging applications. In some embodiments, the sensor component 714 may further include an accelerometer, a gyroscope sensor, a magnetic sensor, a pressure sensor, or a temperature sensor.

The communication component 716 is configured to facilitate wired or wireless communication between the device 700 and other devices. The device 700 may access a wireless network based on a communication standard, such as WiFi, 2G, 5G, or 4G, or a combination thereof. In an exemplary embodiment, the communication component 716 receives broadcast signals or broadcast-related information from an external broadcast management system via a broadcast channel. In an exemplary embodiment, the communication component 716 further includes a near-field communication (NFC) module to facilitate short-range communication. For example, the NFC module may be implemented based on radio-frequency identification (RFID) technology, Infrared Data Association (IrDA) technology, ultra-wideband (UWB) technology, Bluetooth (BT) technology, or other technologies.

In an exemplary embodiment, the device 700 may be implemented by one or more application-specific integrated circuits (ASICs), digital signal processors (DSPs), digital signal processing devices (DSPDs), programmable logic devices (PLDs), field-programmable gate arrays (FPGAs), controllers, micro-controllers, microprocessors, or other electronic components for performing the above-mentioned method.

In an exemplary embodiment, a non-temporary computer-readable storage medium including instructions is further provided, such as the memory 704 including instructions. The above-mentioned instructions may be executed by the processor 720 of the device 700 to complete the above-mentioned method. For example, the non-temporary computer-readable storage medium may be a ROM, a random-access memory (RAM), a CD-ROM, a magnetic tape, a floppy disk, or an optical data storage device.

Those skilled in the art will readily conceive other embodiments of the present disclosure after considering the specification and practicing the embodiments disclosed herein. The present disclosure is intended to cover any variations, uses, or adaptations of the present disclosure that follow the general principles of the present disclosure and include common knowledge or conventional technical means in the technical field not disclosed in the present disclosure. The specification and examples are to be regarded as exemplary only, and the scope and spirit of the present disclosure are indicated by the following claims.

It should be understood that the present disclosure is not limited to the precise structures that are described above and shown in the drawings, and various modifications and changes can be made without departing from its scope. The scope of the present disclosure is limited by the appended claims.

The above are merely preferred embodiments of the present disclosure and are not intended to limit the present disclosure. Any modifications, equivalent replacements, improvements or the like made within the spirit and principle of the present disclosure shall be included in the scope of protection of the present disclosure.

**Claims**

1. A method for action quality evaluation, comprising:

   obtaining (201) motion data of a user collected during a fitness session;
   determining (202) a value of at least one action evaluation metric of the user according to the motion data; and
   determining (203) an action quality evaluation result of the user according to the value of the at least one action evaluation metric;
   wherein the at least one action evaluation metric includes at least one of:

   action consistency, for evaluating at least one of amplitude consistency or completion time consistency of a plurality of fitness actions performed by the user;
   action stability, for evaluating a degree of jitter or wobbling of one or more fitness actions performed by the user;
   action continuity, for evaluating a continuity degree of one or more fitness actions performed by the user;
   action variability, for evaluating an intensity variability of a plurality of fitness actions performed by the user; or
   force control, for evaluating force exertion condition during one or more fitness actions performed by the user.

2. The method according to claim 1, wherein determining the value of the at least one action evaluation metric of the user according to the motion data comprises:

   determining metric feature data for each of at least one fitness action performed by the user according to the motion data; and
   obtaining the value of the at least one action evaluation metric for the user according to the metric feature data for each of the at least one fitness action.

3. The method according to claim 2, wherein the metric feature data for each of the at least one fitness action is determined without taking an exercise type of the each fitness action into consideration; or

> determination of the metric feature data for each of the at least one fitness action comprises performing data reconstruction processing on the motion data, so as to mitigate or eliminate an influence of the exercise type; or
> the metric feature data for each of the at least one fitness action is obtained by performing singular value decomposition processing on the motion data.

4. The method according to any one of claims 1 to 3, wherein determining the value of the at least one action evaluation metric of the user according to the motion data comprises:

> determining the value of the at least one action evaluation metric without taking an exercise type of at least one fitness action performed by the user into consideration; or
> performing data reconstruction processing on the motion data, so as to mitigate or eliminate an influence of the exercise type; or
> performing singular value decomposition processing on the motion data.

5. The method according to any one of claims 1 to 4, wherein determining the value of the at least one action evaluation metric of the user according to the motion data comprises:

> obtaining at least one motion data segment according to the motion data, each motion data segment comprising motion data corresponding to at least one fitness action;
> performing data reconstruction processing on each of the at least one motion data segment to obtain at least one reconstructed data segment; and
> obtaining the value of the at least one action evaluation metric of the user according to the at least one reconstructed data segment.

6. The method according to claim 5, wherein the reconstructed data segment comprises at least one of virtual principal axis data or virtual secondary axis data, wherein the virtual principal axis data is used for representing a signal response to primary motion of the user, and the virtual secondary axis data is used for representing a signal response to sources other than the primary motion.

7. The method according to claim 5 or 6, wherein obtaining the value of the at least one action evaluation metric of the user according to the at least one reconstructed data segment comprises:

> determining a value of at least one first action evaluation metric for one or more fitness actions performed by the user according to virtual principal axis data in the reconstructed data segment, the at least one first action evaluation metric comprising at least one of action consistency, action stability, action continuity, action variability or action regularity; or
> determining a value of at least one second action evaluation metric for one or more fitness actions performed by the user according to virtual secondary axis data in the reconstructed data segment, the at least one second action evaluation metric comprising at least one of action stability or force control.

8. The method according to any one of claims 1 to 7, wherein the action quality evaluation result of the user comprises at least one of:

> a statistical result of the value of the at least one action evaluation metric of the user;
> evaluative description of the at least one action evaluation metric of the user; or
> an action improvement suggestion for the user.

9. A method for action quality evaluation, comprising:

> obtaining (401) motion data of a user collected during a fitness session;
> obtaining (402) at least one motion data segment according to the motion data, wherein each motion data segment comprises motion data corresponding to at least one fitness action;
> performing (403) data reconstruction processing on each of the at least one motion data segment to obtain at least one reconstructed data segment; and
> obtaining (404) an action quality evaluation result of the user according to the at least one reconstructed data segment.

10. The method according to claim 9, wherein:

> the data reconstruction processing is used to mitigate or eliminate an influence of an exercise type of the at least one fitness action; or
> the data reconstruction processing comprises singular value decomposition processing; or
> the reconstructed data segment comprises at least one of virtual principal axis data or virtual secondary axis data, wherein the virtual principal axis data is used for representing a signal response to primary motion of the user, and the virtual secondary axis data is used for representing a signal response to sources other than the primary motion.

**11.** The method according to claim 9 or 10, wherein obtaining the action quality evaluation result of the user according to the at least one reconstructed data segment comprises:

determining a value of at least one action evaluation metric for each of the at least one motion data segment according to the at least one reconstructed data segment; and
determining the action quality evaluation result of the user according to the value of the at least one action evaluation metric for each of the at least one motion data segment.

**12.** The method according to any one of claims 9 to 11, wherein obtaining the action quality evaluation result of the user according to the at least one reconstructed data segment comprises:

determining a value of at least one first action evaluation metric for one or more fitness actions associated with each of the at least one motion data segment according to virtual principal axis data in the reconstructed data segment corresponding to the motion data segment, wherein the at least one first action evaluation metric comprises at least one of action consistency, action stability, action continuity, action variability or action regularity; or
determining a value of at least one second action evaluation metric for one or more fitness actions associated with each of the at least one motion data segment according to virtual secondary axis data in the reconstructed data segment corresponding to the motion data segment, wherein the at least one second action evaluation metric comprises at least one of action stability or force control.

**13.** An apparatus for action quality evaluation, comprising:

at least one unit or module for executing the method according to any one of claims 1 to 8 or the method according to any one of claims 9 to 12.

**14.** A computer-readable storage medium having a computer program stored thereon, wherein the computer program, when executed by a processor, causes the processor to implement the method according to any one of claims 1 to 8 or the method according to any one of claims 9 to 12.

**15.** An electronic device, comprising:

a processor; and
a memory, configured to store instructions executable by the processor;
wherein the processor is configured to execute the method according to any one of claims 1 to 8 or the method according to any one of claims 9 to 12.

FIG. 1

motion data collected during a fitness session is obtained $\sim$201

a value of at least one action evaluation metric of the user is determined based on the motion data $\sim$202

an action quality evaluation result of the user is determined according to the value of the at least one action evaluation metric $\sim$203

FIG. 2

FIG. 3

motion data of the user collected during the fitness session is acquired ⟋401

↓

at least one motion data segment is obtained based on the motion data, where each motion data segment includes motion data corresponding to at least one fitness action ⟋402

↓

data reconstruction processing is performed on each of the at least one motion data segment to obtain at least one reconstructed data segment ⟋403

↓

an action quality assessment result of the user is obtained based on the at least one reconstructed data segment ⟋404

FIG. 4

apparatus for action quality assessment

⟋ 51

motion data acquisition module

⟋ 52

metric value determination module

⟋ 53

first assessment result determination module

FIG. 5

apparatus for action quality assessment

motion data acquisition module — 51

motion data segment determination module — 62

reconstructed data segment determination module — 63

second assessment result determination module — 64

FIG. 6

<u>700</u>

storage — 704

processing component — 702

power supply — 706

processor — 720

multimedia — 708

audio — 710

communication component — 716

sensor component — 714

Input/output interface — 712

FIG. 7

Europäisches
Patentamt

European
Patent Office

Office européen
des brevets

# EUROPEAN SEARCH REPORT

**Application Number**

EP 25 17 7590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/111257 A1 (TSUR GUY [IL] ET AL) 14 April 2022 (2022-04-14) * paragraphs [0225] - [0232], [0246], [0327], [0355] - [0356] * ----- | 1-15 | INV. G16H20/30 A61B5/11 |
| A | US 2022/296966 A1 (ASIKAINEN SAMI [CA] ET AL) 22 September 2022 (2022-09-22) * paragraphs [0078], [0089] * ----- | 1-15 | |

**TECHNICAL FIELDS SEARCHED (IPC)**

G16H
A61B

The present search report has been drawn up for all claims

| Place of search | Date of completion of the search | Examiner |
|---|---|---|
| Munich | 10 October 2025 | Reinbold, Bernhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

..................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 17 7590

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2025

| Patent document cited in search report | Publication date | Patent family member(s) | Publication date |
|---|---|---|---|
| US 2022111257 A1 | 14-04-2022 | NONE | |
| US 2022296966 A1 | 22-09-2022 | NONE | |

EPO FORM P0459

For more details about this annex : see Official Journal of the European Patent Office, No. 12/82